(19) Europäisches Patentamt
European Patent Office
Office européen des brevets

(11) **EP 2 712 673 A1**

(12) **EUROPÄISCHE PATENTANMELDUNG**

(43) Veröffentlichungstag:
**02.04.2014 Patentblatt 2014/14**

(21) Anmeldenummer: **12186486.2**

(22) Anmeldetag: **28.09.2012**

(51) Int Cl.:
*B01J 21/08* (2006.01)      *B01J 21/10* (2006.01)
*B01J 21/14* (2006.01)      *B01J 23/22* (2006.01)
*B01J 23/28* (2006.01)      *B01J 23/30* (2006.01)
*B01J 23/34* (2006.01)      *B01J 23/56* (2006.01)
*B01J 23/72* (2006.01)      *B01J 23/755* (2006.01)
*B01J 23/78* (2006.01)      *C07C 11/167* (2006.01)
*C07C 1/02* (2006.01)      *C07C 1/20* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **LANXESS Deutschland GmbH
50569 Köln (DE)**

(72) Erfinder:
• **Haufe, Rommy
57439 Attendorn (DE)**
• **Reschetilowski, Wladimir
01445 Radebeul (DE)**
• **Bernhard, Maik
01219 Dresden (DE)**

(54) **Verfahren zur Herstellung von 1,3-Butadien**

(57) Die vorliegende Erfindung betrifft ein Verfahren zur Herstellung von 1,3-Butadien ausgehend von Ethanol an Sauerstoffverbindungen des Magnesiums und Siliciums enthaltenden Katalysatoren sowie die Katalysatoren selbst.

EP 2 712 673 A1

**Beschreibung**

Die vorliegende Erfindung betrifft ein Verfahren zur Herstellung von 1,3-Butadien ausgehend von Ethanol an Sauerstoffverbindungen des Magnesiums und Siliciums enthaltenden Katalysatoren sowie die Katalysatoren selbst.

Die Herstellung von 1,3-Butadien aus Ethanol in Gegenwart anorganischer Katalysatoren hat insbesondere vor dem Hintergrund der Möglichkeit, Ethanol aus nicht-fossilen Quellen zu gewinnen in den vergangenen Jahren wieder besonderes Interesse geweckt.

Basierend auf den frühen Arbeiten von Lebedev (GB 331482, 1929) wurde eine Vielzahl von Katalysatorsystemen entwickelt. Insbesondere solche, die Sauerstoffverbindungen des Magnesiums und Siliciums sowie gegebenenfalls Dotierungen mit anderen Metalloxiden wie Übergangsmetalloxiden aufweisen, haben sich als relativ selektiv erwiesen.

So sind beispielsweise aus Makshina, E.V; Janssens, W.; Sels, B.F; Jacobs, P.A., "Catalytic study of the conversion of ethanol into 1,3-butadiene", Catalysis Today, 2012 übergangsmetall- dotierte Katalysatoren auf Basis von Magnesiumoxid und Siliciumdioxid bekannt, die bei niedrigen Beladungen Selektivitäten bis zu 63 % erreichen.

Erdev et al., Acta Chimica, Academiae Scientiarium Hungaricae Tomus 50, 1966, 163 - 166 beschreiben den Einfluss von Zink-Dotierung bei Magnesiumoxid-Katalysatoren.

Aus PL 44585 (1961) sind zink-dotierte Katalysatoren auf Basis von Magnesiumoxid, Siliciumdioxid und Kaolin bekannt, die eine Selektivität von etwa 69 % aufweisen.

Aus Ohnishi et al., J. Chem. Soc., Chem. Commun. 1985, 1613-1614 sind alkalimetall-dotierte Katalysatoren auf Basis von Magnesiumoxid und Siliciumdioxid bekannt, die Selektivitäten bis zu 87 % erreichen sollen, allerdings bei nicht vollständigem Umsatz. Eigene Versuche haben jedoch gezeigt, dass diese Werte nicht erreicht werden können.

Aus Y. Kitayama, K. Shimizu T. Kodama S. Murai T. Mizusima M. Hayakawaa and M. Muraoka "Role of intracrystalline tunnels of sepiolite for catalytic activity", Studies in Surface Science and Catalysis 2002, 142, 675-682, ist der Einsatz von Sepioliten, einem Magnesium und Silicium haltigen natürlichen Mineral, bekannt, in denen die Magnesiumatome des Kristallgitters teilweise durch Übergangsmetallatome ausgetauscht wurden. Für einen mit Zink ausgetauschten Sepiolith wurden Selektivitäten von bis zu 67 % beschrieben, bei gleichzeitiger Anwendung von gasförmigem Ammoniak sogar 71 %.

Schließlich werden in WO 2012/015340 A weitere Katalysatoren auf Basis von Magnesiumoxid und Siliciumdioxid beschrieben, die Selektivitäten von 40 % aufweisen.

Allen bislang bekannten Verfahren ist gemeinsam, dass hohe Selektivitäten zu 1,3-Butadien bei gleichzeitig hoher Katalysatorbeladung nicht in kommerziell attraktivem Ausmaß erreicht werden können. Es bestand daher die Aufgabe Katalysatoren und ein Verfahren unter ihrer Verwendung bereitzustellen, das die Nachteile des Standes der Technik überwindet.

Es wurde nun ein Katalysator gefunden, enthaltend Sauerstoffverbindungen des Magnesiums und des Siliciums, dadurch gekennzeichnet, dass das molare Verhältnis von Magnesium zu Silicium von 3,5:1 bis 15:1, vorzugsweise 4:1 bis 15 :1 und besonders bevorzugt 5,5:1 bis 12,5:1 beträgt.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

Unter Sauerstoffverbindungen des Magnesiums sind solche Verbindungen zu verstehen in denen Sauerstoff entweder als Oxid-Ion, Hydroxidion oder kovalent an Magnesium gebunden vorliegt.

Unter Sauerstoffverbindungen des Siliciums sind solche Verbindungen zu verstehen, in denen Sauerstoff als Oxid-Ion, Hydroxidion oder kovalent an Silicium gebunden vorliegt.

Weiterhin werden unter dem Begriff Sauerstoffverbindungen des Magnesiums und des Siliciums auch Magnesium enthaltende Silikate subsumiert.

Dem Fachmann ist dabei klar, dass der Bindungscharakter von Magnesium-Sauerstoff-Bindungen nach dem Pauling-Modell der Elektronegativitätsdifferenz nicht ausschließlich aber überwiegend ionisch ist, während Silicium-Sauerstoff-Bindungen nicht ausschließlich aber eher kovalenten Charakter besitzen. Die oben genannten Definitionen schließen diese Mischformen mit ein.

Bevorzugte Sauerstoffverbindungen des Magnesiums sind Magnesiumhydroxid und Magnesiumoxid, wobei Magnesiumoxid bevorzugt ist. Besonders bevorzugt ist kristallines Magnesiumoxid.

Bevorzugte Sauerstoffverbindungen des Siliciums sind alle kristallinen oder nicht-kristallinen Modifikationen bzw. Erscheinungsformen des Siliciumdioxids und Kieselsäuren der Formel $H_{2n+2}Si_nO_{3n+1}$, wobei n eine natürliche Zahl ist.

Besonders bevorzugt sind pyrogene Kieselsäuren, wobei unter pyrogenen Kieselsäuren amorphe Siliciumdioxid-Pulver zu verstehen sind, die eine spezifische Oberfläche von 50 bis 600 m$^2$/g aufweisen. Soweit nicht anders angegeben, werden die in dieser Anmeldung angegebenen spezifische Oberflächen bestimmt nach DIN 66132.

Ebenfalls geeignete Sauerstoffverbindungen des Magnesiums und Siliciums sind Magnesiumsilikate, wie zum Beispiel Talk und Sepiolith sowie Mineralien der Serpentingruppe. In einer bevorzugten Ausführungsform enthalten die erfindungsgemäßen Katalysatoren als Sauerstoffverbindungen des Siliciums pyrogene Kieselsäure sowie als Sauerstoffverbindung des Magnesiums Magnesiumoxid.

In einer weiteren Ausführungsform enthalten die erfindungsgemäßen Katalysatoren von 70 bis 100 Gew.-% Sauerstoffverbindungen des Magnesiums und des Siliciums, bevorzugt 90 bis 100 Gew.-%, besonders bevorzugt 98 bis 100 Gew.-%.

In einer weiteren Ausführungsform enthalten die erfindungsgemäßen Katalysatoren weiterhin zumindest eine Übergangsmetallverbindung, wobei die Übergangsmetallverbindungen vorzugsweise ausgewählt sind aus Sauerstoff-Verbindungen der Elemente Titan, wie insbesondere Titan(IV)oxid, Vanadium, wie insbesondere Vanadium(V)oxid, Molybdän, Wolfram, Mangan, wie insbesondere Mangan(II)oxid, Kupfer, wie insbesondere Kupfer(II)oxid, Nickel, wie insbesondere Nickel(II)oxid, Zink, wie insbesondere Zink(II)oxid oder Chrom, wie insbesondere Chrom(III)oxid.

Bevorzugt sind Sauerstoff-Verbindungen der Elemente Titan, Mangan, Kupfer, Nickel, Zink oder Chrom, wobei Sauerstoff-Verbindungen des Zinks und Nickels, wobei insbesondere Zink(II)oxid und Nickel(II)oxid noch weiter bevorzugt sind und ganz besonders Zink(II)oxid bevorzugt ist.

In einer Ausführungsform enthalten die erfindungsgemäßen Katalysatoren zwischen 70 und 100 Gew.-% Sauerstoffverbindungen des Magnesiums und des Siliciums, sowie mindestens einer Übergangsmetallverbindung, bevorzugt 90 bis 100 Gew.-%, besonders bevorzugt 99 bis 100 Gew.-% und ganz besonders bevorzugt 99,9 bis 100,0 Gew.-%.

In diesem Fall beträgt das molare Verhältnis von Magnesium zu Übergangsmetall von 15:1 bis 1000:1, vorzugsweise 50:1 bis 750:1, besonders bevorzugt von 75:1 bis 500:1 und ganz besonders bevorzugt von 80:1 bis 350:1.

Die Differenz zu 100 Gew.-% können beispielsweise Sauerstoffverbindungen anderer Metalle als Magnesium oder alternativ oder zusätzlich Inertstoffe sein, die unter den Reaktionsbedingungen nicht mit Edukten oder Produkten reagieren. Solche Inertstoffe können beispielsweise Graphit, Siliciumcarbid oder Bornitrid sein.

In einer Ausführungsform enthalten die erfindungsgemäßen Katalysatoren 0,1 Gew.-% oder weniger an Alkalimetallverbindungen berechnet auf $M_2O$ wobei M ein Alkalimetall, wie insbesondere Lithium, Natrium oder Kalium ist, vorzugsweise weniger als 0,08 Gew.-%.

In einer Ausführungsform beträgt die spezifische Oberfläche der erfindungsgemäßen Katalysatoren 20 bis 500 $m^2/g$, vorzugsweise 20 bis 250 $m^2/g$ und besonders bevorzugt 100 bis 250 $m^2/g$.

In einer weiteren Ausführungsform beträgt das Porenvolumen gemessen nach DIN 66 133 von 0,05 bis 0,4 $cm^3/g$, vorzugsweise 0,1 bis 0,4 $cm^3/g$ und besonders bevorzugt 0,13 bis 0,34 $cm^3/g$.

Die Herstellung der erfindungsgemäßen Katalysatoren erfolgt beispielsweise in einem Verfahren, das zumindest folgende Schritte umfasst:

A) Herstellung eines Katalysatormaterials durch

i) Fällung zumindest einer im Wesentlichen wasserlöslichen Magnesiumverbindung auf zumindest eine feste Sauerstoffverbindung des Siliciums oder zumindest einer nicht festen Siliciumverbindung auf zumindest eine feste Sauerstoffverbindung des Magnesiums und/oder Magnesiumcarbonat in einem wässrigen Reaktionsmedium und Abtrennung des so erhaltenen, gefällten Katalysatormaterials vom wässrigen Reaktionsmedium

ii) Bereitstellung von Mischungen von Sauerstoffverbindungen des Magnesiums oder Magnesiumcarbonat und Sauerstoffverbindungen des Siliciums als Katalysatormaterial oder Bereitstellung von Sauerstoffverbindungen des Magnesiums und des Siliciums jeweils gemäß obiger Definition als Katalysatormaterial

B) Gegebenenfalls Vermahlung des Katalysatormaterials

C) Gegebenenfalls Herstellung von Formkörpern enthaltend das gemäß Schritt A) und/oder B) erhaltene Katalysatormaterial

D) Kalzinierung des Katalysatormaterials oder der Formkörper

Gemäß Schritt A) wird ein Katalysatormaterial durch Fällung in einem wässrigen Reaktionsmedium gewonnen.

Der Begriff "wässriges Reaktionsmedium" bedeutet im Rahmen der Erfindung ein flüssiges, Wasser enthaltendes Medium, das vorzugsweise mindestens 80 Gew.-% Wasser enthält und besonders bevorzugt frei von organischen Lösungsmitteln ist, die nicht aus den eingesetzten Magnesium- oder Siliciumverbindungen stammen.

Der Begriff "Fällung" in einem wässrigen Reaktionsmedium umfasst die Konvertierung von im Wesentlichen wasserlöslichen Magnesiumverbindungen oder nicht festen Siliciumverbindungen zu nicht löslichen Sauerstoffverbindungen des Magnesiums oder Magnesiumcarbonat bzw. nicht löslichen Sauerstoffverbindungen des Siliciums.

Nicht feste Siliciumverbindungen sind entweder wasserlösliche Siliciumverbindungen oder Siliciumverbindungen, die einen Schmelzpunkt von weniger als 20°C bei 1013 hPa aufweisen.

Im Wesentlichen wasserlösliche Magnesiumverbindungen und Siliciumverbindungen sind solche, die bei 20°C und einem Druck von 1013 hPa eine Wasserlöslichkeit von mindestens 5,0 g/l aufweisen.

Im Wesentlichen wasserunlösliche Magnesium- und Siliciumverbindungen sind dementsprechend solche, die bei 20°C und einem Druck von 1013 hPa eine Wasserlöslichkeit von weniger als 5,0 g/l aufweisen.

Geeignete, im Wesentlichen wasserlösliche Magnesiumverbindungen sind beispielsweise Magnesiumnitrat, -sulfat, -chlorid, -bromid, -acetat, oder deren Hydrate oder Mischungen der vorgenannten Verbindungen.

Wasserlösliche Siliciumverbindungen sind zum Beispiel Wassergläser, Siliciumverbindungen, die einen Schmelzpunkt von weniger als 20°C bei 1013 hPa aufweisen sind beispielsweise Tetraethylorthosilicat und Tetramethylorthosilicat und Tetraisopropylorthosilicat.

Für Schritt A verwendete feste Sauerstoffverbindungen des Magnesiums und/oder Siliciums können beispielsweise sein:

Magnesiumoxid, Magnesiumcarbonat und Magnesiumhydroxid, die oben genannten Sauerstoffverbindungen des Siliciums oder die oben genannten Magnesiumsilicate.

Die Fällung von Magnesiumverbindungen erfolgt beispielsweise derart, dass der pH-Wert des wässrigen Reaktionsmediums bei der Konvertierungstemperatur durch Zugabe von Basen auf über 7,0 bis 12,0, vorzugsweise 7,5 bis 9,0 und besonders bevorzugt 7,7 bis 8,5 eingestellt wird.

Die Fällung von Siliciumverbindungen erfolgt vorzugsweise derart, dass der pH-Wert des wässrigen Reaktionsmediums bei der Konvertierungstemperatur auf 2 bis 12 eingestellt wird.

Die Temperatur, bei der Fällung erfolgt (Konvertierungstemperatur) beträgt beispielsweise 0 bis 100°C, vorzugsweise 20 bis 85°C und besonders bevorzugt 50 bis 80°C.

Geeignete Basen sind vorzugsweise solche, die in Wasser einen $pK_B$-Wert bei Standardbedingungen von 5,0 oder weniger, vorzugsweise 4,0 oder weniger aufweisen. Beispielhaft seien Alkalimetallcarbonate und -hydroxide wie beispielsweise Lithium-, Natrium- oder Kaliumcarbonat sowie Lithium-, Natrium oder Kaliumhydroxid genannt.

Im Schritt A) i) erfolgt die Abtrennung des gemäß Schritt A) erhaltenen, gefällten Katalysatormaterials vom wässrigen Reaktionsmedium.

Die Abtrennung kann beispielsweise durch Filtration, Zentrifugation oder Sedimentation und Dekantieren oder andere dem Fachmann bekannten Fest-Flüssig-Trennoperationen erfolgen, wobei Filtration bevorzugt ist.

Gemäß Schritt B) kann optional, aber bevorzugt die Vermahlung erfolgen.

Die Vermahlung kann in an sich bekannter Weise beispielsweise durch eine Kugelmühle oder einen Mixer erfolgen. Typische Zeiträume sind beispielsweise 5 min bis 72 Stunden, vorzugsweise 1 Stunde bis 24 Stunden.

Gemäß Schritt C) kann optional die Herstellung von Formkörpern erfolgen. Schritt C) kann auch nach Schritt D) erfolgen oder aber nach Schritt A) bzw. B).

Die Herstellung der Formkörper kann in an sich bekannter Weise beispielsweise durch Tablettierung oder Extrudierung oder andere, dem Fachmann bekannte Methoden erfolgen.

Im Schritt D) erfolgt die Kalzinierung.

Im Rahmen der Erfindung ist unter dem Begriff "Kalzinierung" eine thermische Behandlung des Katalysatormaterials oder der Formkörper auf eine Temperatur von 250 bis 1500°C, vorzugsweise 300 bis 1000°C zu verstehen.

Die Kalzinierung kann beispielsweise für einen Zeitraum von 30 min bis 120 h Stunden erfolgen, vorzugsweise 2 h bis 12 h. Längere Kalzinierungen zeigen typischerweise keinen Effekt.

In einer Ausführungsform der Erfindung kann das Kalzinieren im Reaktor durchgeführt werden, der für das erfindungsgemäße Verfahren eingesetzt wird.

Beim Kalzinieren kann teilweise eine Gewichtsverlust beobachtet werden, der typischerweise auf Eliminierungsreaktionen von Wasser aus Hydroxyverbindungen wie Magnesiumhydroxid oder Kieselsäuren oder Kohlendioxid aus Carbonaten zurückzuführen ist.

Sofern die erfindungsgemäßen Katalysatoren Übergangsmetallverbindungen, vorzugsweise ausgewählt aus Sauerstoff-Verbindungen der Elemente Titan, Vanadium, Molybdän, Wolfram, Mangan, Kupfer, Nickel, Zink oder Chrom enthalten, können diese beispielsweise im Schritt A durch Co-Fällung von wasserlöslichen Übergangsmetallverbindungen mit den wasserlöslichen Magnesiumverbindungen eingebracht werden, wobei typischerweise die entsprechenden Oxide oder Hydroxide entstehen, oder direkt beispielsweise in Form von Oxiden, Hydroxiden, Nitraten oder Carbonaten im Schritt A ii) der Mischung zugesetzt werden.

Beispielsweise lassen sich die erfindungsgemäßen Katalysatoren wie folgt herstellen:

A) Herstellung eines Katalysatormaterials durch Vermischung von

● Kieselgelen oder pyrogener Kieselsäure oder Mischungen davon, vorzugsweise pyrogener Kieselsäure mit

- Magnesiumhydroxid, Magnesiumoxid oder Magnesiumcarbonat, vorzugsweise Magnesiumhydroxid und

- gegebenenfalls Übergangsmetallverbindungen wie vorzugsweise Übergangsmetalloxiden, -hydroxiden, -nitraten oder -carbonaten, vorzugsweise Oxide, Hydroxide, Nitrate oder Carbonate von Titan, Vanadium, Molybdän, Wolfram, Mangan, Kupfer, Nickel, Zink oder Chrom, besonders bevorzugt die Hydroxide und Oxide von Titan, Vanadium, Mangan, Kupfer, Nickel, Zink oder Chrom sowie Mangan, Zink-, Nickel und Kupfercarbonat

B) Vermahlung des gemäß Schritt A) erhaltenen Katalysatormaterials
C) Gegebenenfalls Herstellung von Formkörpern enthaltend das gemäß Schritt A) und/oder B) erhaltene Katalysatormaterial
D) Kalzinierung des Katalysatormaterials oder der Formkörper

In einem Aspekt betrifft die Erfindung auch Katalysatoren erhältlich nach dem erfindungsgemäßen Verfahren.

Die erfindungsgemäßen Katalysatoren und erfindungsgemäß erhältlichen Katalysatoren eignen sich insbesondere zum Einsatz in einem Verfahren zur Herstellung von 1,3-Butadien aus Ethanol.

In einer Ausführungsform des erfindungsgemäßen Verfahrens erfolgt die Herstellung von 1,3-Butadien durch Inkontaktbringen eines Ethanol enthaltenden Gasstromes mit erfindungsgemäßem Katalysator.

Das Ethanol im Ethanol enthaltenden Gasstrom kann einen Partialdruck von beispielsweise 0,01 bis 100 % bezogen auf den Gesamtdruck des Gasstromes beitragen, vorzugsweise 0,1 bis 100 %, besonders bevorzugt 0,1 bis 10 %.

Der Gasstrom kann weiterhin Inertgase enthalten, wobei unter Inertgasen solche Gase zu verstehen sind, die bei den Reaktionsbedingungen nicht oder nicht messbar reagieren. Beispiele für Inertgase sind Edelgase wie insbesondere Argon sowie andere Gase wie insbesondere Stickstoff.

Der Gasstrom kann in einer Ausführungsform gegebenenfalls weiterhin Wasser enthalten, wobei in diesem Fall der Wassergehalt im Gasstrom bezogen auf Ethanol beispielsweise mehr als 0 bis 10 Gew.-% beträgt, vorzugsweise 0,5 bis 5 und besonders bevorzugt eine Menge die dem Ethanol-Wasser Azeotrop bei den gewählten Reaktionsbedingungen entspricht.

In einer anderen Ausführungsform beträgt der Wassergehalt im Gasstrom bezogen auf Ethanol beispielsweise 10.000 Gew.-ppm oder weniger, vorzugsweise 1.000 Gew.-ppm oder weniger.

Der Ethanol enthaltende Gasstrom kann beispielsweise durch Kontaktieren, insbesondere Überströmen oder Einleiten eines Inertgasstromes über bzw. in Ethanol erfolgen, wobei über die Temparatur die Sättigung bzw. der Gehalt des Ethanols im Ethanol enthaltenden Gasstrom eingestellt werden kann. Alternativ ist simples Verdampfen von Ethanol möglich.

Die Temperatur beim Inkontaktbringen eines Ethanol enthaltenden Gasstromes mit erfindungsgemäßem Katalysator kann beispielsweise von 200°C bis 500°C betragen, vorzugsweise 250°C bis 475°C, weiter bevorzugt 325°C bis 475°C und besonders bevorzugt 350 bis 475°C.

Der Druck beim Inkontaktbringen kann beispielsweise 100 hPa bis 2 MPa betragen, 900 hPa bis 1200 hPa, insbesondere Umgebungsdruck ist besonders bevorzugt.

Das Verfahren kann batchweise oder kontinuierlich betrieben werden. In einer bevorzugten Ausführungsform wird das Verfahren kontinuierlich betrieben.

Erfindungsgemäß wird ein Produktgasgemisch erhalten, das 1,3-Butadien, gegebenenfalls unumgesetztes Ethanol sowie Nebenprodukte wie beispielsweise Ethylen und Aceton enthält.

Die Abtrennung von 1,3-Butadien aus dem Produktgasgemisch kann beispielsweise durch Ausfrieren oder andere an sich bekannte thermische oder selektiv adsorptive Trennverfahren erfolgen.

In einer Ausführungsform des erfindungsgemäßen Verfahrens wird die Katalysatorbeladung so gewählt, dass sie mehr als 0,25, vorzugsweise 0,5 bis 10,0 und besonders bevorzugt 1,0 bis 5,0 g Ethanol / g Katalysator beträgt.

In einer weiteren Ausführungsform des erfindungsgemäßen Verfahrens werden Reaktionstemperatur, Druck, Beladung und Verweilzeit so gewählt, dass mehr als 50 % des eingesetzten Ethanols umgesetzt werden, vorzugsweise mehr als 70 % und besonders bevorzugt mehr als 80 %.

Gegebenenfalls nicht umgesetztes Ethanol kann erneut in die Reaktion zurückgeführt werden.

Die Einsatzzeit der Katalysatoren, insbesondere in kontinuierlichen Verfahren beträgt typischerweise 5 Stunden oder mehr.

Der besondere Vorteil der Erfindung ist darin zu sehen, dass Katalysatoren bereitgestellt werden, die bei hoher Katalysatorbeladung gleichzeitig gute Selektivitäten erlauben und somit eine 1,3-Butadienerzeugung von bis zu 0,8 [g/g Katalysator *h] ermöglichen.

Die Reaktionsführung wird anhand der Beispiele näher erläutert.

## Beispiele

## Allgemeine Vorschrift zur Katalysatorherstellung

Als Sauerstoffverbindung des Siliciums wurde pyrogene Kieselsäure des Typs HDK® N20 der Fa. Wacker, nachstehend HDK genannt, eingesetzt. Sie wies folgende Spezifikation auf.

| Eigenschaft | Einheit | Wert |
|---|---|---|
| spezifische Oberfläche | $[m^2/g]$ | 233 |
| Porenvolumen | $[cm^3/g]$ | 0,31 |
| Partikelgrößenverteilung | 10 % aller Partikel (Anzahl) | 8 $\mu$m |
|  | 50 % aller Partikel | 38 $\mu$m |
|  | 90 % aller Partikel | 91 $\mu$m |
| Schmelzpunkt | [°C] | 1700 |
| Dichte (DIN 51757) | $[g/cm^3]$ | 2,2 |
| Schüttdichte | $[kg/m^3]$ | 20 - 130 |
| pH-Wert (DIN EN ISO 787-9) | [-] | 3,6 - 4,5 |

## Herstellung von Katalysatoren durch Vermahlung in der Kugelmühle

Zur Herstellung des Katalysators gemäß Beispiel 1 (MgO-HDK-24k) wurden 4,296 g (0,0737 mol) $Mg(OH)_2$ und 0,774 g (0,0129 mol) HDK in einem Becherglas eingewogen. Das Pulver wurde anschließend in eine Kugelmühle überführt und 24 h kontinuierlich gemahlen. Anschließend erfolgte eine Kalzination für 6h bei 500°C im Stickstoffstrom (10 l/h). Dabei wurde ein Katalysator enthaltend 85 Mol-% MgO und 15 Mol % $SiO_2$ in Form von HDK erhalten.

Analog zu Beispiel 1 wurden im Beispiel 11 4,296 g (0,0736 mol) $Mg(OH)_2$ und 0,352 g (0,0060 mol) HDK eingesetzt. Dabei wurde ein Katalysator enthaltend 92,5 Mol-% MgO und 7,5 Mol % $SiO_2$ in Form von HDK erhalten.

## Herstellung von Katalysatoren durch Aufschlämmung und Trocknung mit anschließender Mahlung in der Kugelmühle

Zur Herstellung des Katalysators gemäß Beispiel 2 (MgO-HDK-ZnO-0,25-24ki) wurden 7,8705 g (0,135 mol) $Mg(OH)_2$, 1,4424 g (0,024 mol) HDK und 0,0326 g (0,0004mol) Zinkoxid in einem Becherglas eingewogen. Für eine bessere Homogenisierung wurde die Probe, entsprechend des Schüttvolumens der Einwaage mit dem 2,5-fachen Volumen an entionisiertem Wasser versetzt und dieses am Rotationsverdampfer bei 72°C und 172 mbar abgezogen. Anschließend wurde die Probe über Nacht bei 60°C getrocknet und in die Kugelmühle überführt. Der letzte Präparationsschritt umfasste eine Kalzination für 6h bei 500°C im Stickstoffstrom (10 l/h). Dabei wurde ein Katalysator enthaltend 84,75 Mol-% MgO und 15 Mol % $SiO_2$ in Form von HDK und 0,25 Mol-% ZnO erhalten.

Analog zu Beispiel 2 wurden die Beispiele 3 bis 9 unter Verwendung der folgenden Mengen an Magnesiumhydroxid, HDK und Übergangsmetallverbindungen hergestellt:

**Beispiel 3:** 8,382 g (0,1437 mol) $Mg(OH)_2$, 1,533 g (0,0255 mol) HDK, 0,068g (0,00084 mol) ZnO. Dabei wurde ein Katalysator enthaltend 84,5 Mol-% MgO, 15 Mol % $SiO_2$ in Form von HDK und 0,5 Mol-% ZnO erhalten.

**Beispiel 4:** 5,643 g (0,0968 mol) $Mg(OH)_2$, 1,081 g (0,018 mol) HDK, 0,497 g (0,0061 mol) ZnO. Dabei wurde ein Katalysator enthaltend 80 Mol-% MgO, 15 Mol % $SiO_2$ in Form von HDK und 5 Mol-% ZnO erhalten.

**Beispiel 5:** 8,382 g (0,1437 mol) $Mg(OH)_2$, 1,533 g (0,0255 mol) HDK, 0,206g (0,00084 mol) $Mn(CH_3COO)_2*4H_2O$. Dabei wurde ein Katalysator enthaltend 84,5 Mol-% MgO, 15 Mol % $SiO_2$ in Form von HDK und 0,5 Mol-% MnO erhalten

**Beispiel 6:** 8,379 g (0,1437 mol) $Mg(OH)_2$, 1,533 g (0,0255 mol) HDK, 0,0819g (0,00084 mol) $Cu(OH)_2$. Dabei wurde ein Katalysator enthaltend 84,5 Mol-% MgO, 15 Mol % $SiO_2$ in Form von HDK und 0,5 Mol-% CuO erhalten

**Beispiel 7:** 8,379 g (0,1437 mol) $Mg(OH)_2$, 1,533 g (0,0255 mol) HDK, 0,2443g (0,00084 mol) $Ni(NO_3)_2*6H_2O$. Dabei wurde ein Katalysator enthaltend 84,5 Mol % $SiO_2$ in Form von HDK und 0,5 Mol-% NiO erhalten

**Beispiel 8:** Beispiel 7: 8,379 g (0,1437 mol) $Mg(OH)_2$, 1,533 g (0,0255 mol) HDK, 0,0983g (0,00084 mol) $NH_4VO_3$. Dabei wurde ein Katalysator enthaltend 84,5 Mol-% MgO, 15 Mol % $SiO_2$ in Form von HDK und 0,5 Mol-% $V_2O_5$ erhalten.

**Beispiel 9:** Beispiel 7: 8,379 g (0,1437 mol) $Mg(OH)_2$, 1,533 g (0,0255 mol) HDK, 0,067g (0,00084 mol) Titandioxid. Dabei wurde ein Katalysator enthaltend 84,5 Mol-% MgO, 15 Mol % $SiO_2$ in Form von HDK und 0,5 Mol-% $TiO_2$ erhalten.

**Beispiel 10:** Beispiel 7: 8,379 g (0,1437 mol) $Mg(OH)_2$, 1,533 g (0,0255 mol) HDK, 0,1276g (0,00084 mol) $Cr_2O_3$. Dabei wurde ein Katalysator enthaltend 84,5 Mol-% MgO, 15 Mol % $SiO_2$ in Form von HDK und 0,5 Mol-% $Cr_2O_3$ erhalten.

**Eigenschaften der Katalysatoren**

| Beispiel | Katalysatorprobe | spezifische Oberfläche [$m^2$/g] | Porenvolumen [$cm^3$/g] |
|---|---|---|---|
| 1 | MgO-HDK-24k | 28 | 0,06 |
| 2 | MgO-HDK-ZnO-0,25-24ki | 104 | 0,14 |
| 3 | MgO-HDK-ZnO-0,5-24ki | 112 | 0,12 |
| 4 | MgO-HDK-ZnO-5-24ki | nicht bestimmt | nicht bestimmt |
| 5 | MgO-HDK-MnO-0,5-24ki | 188 | 0,22 |
| 6 | MgO-HDK-CuO-0,5-24ki | 210 | 0,27 |
| 7 | MgO-HDK-NiO-0,5-24ki | 85 | 0,33 |
| 8 | MgO-HDK-$V_2O_5$-0,5-24ki | 223 | 0,27 |
| 9 | MgO-HDK-$TiO_2$-0,5-24ki | 212 | 0,23 |
| 10 | MgO-HDK-$Cr_2O_3$-0,5-24ki | 137 | 0,24 |
| 11 | MgO-HDK-24k (92,5:7,5) | nicht bestimmt | nicht bestimmt |
| 12* | MgO-$SiO_2$(1:1)-0,1Na | nicht bestimmt | nicht bestimmt |
| * zum Vergleich: hergestellt gemäß Ohnishi et al., J. Chem. Soc., Chem. Commun. 1985, 1613-1614 | | | |

**Allgemeine Vorschrift zur Durchführung der katalytischen Tests**

**Versuchsaufbau**

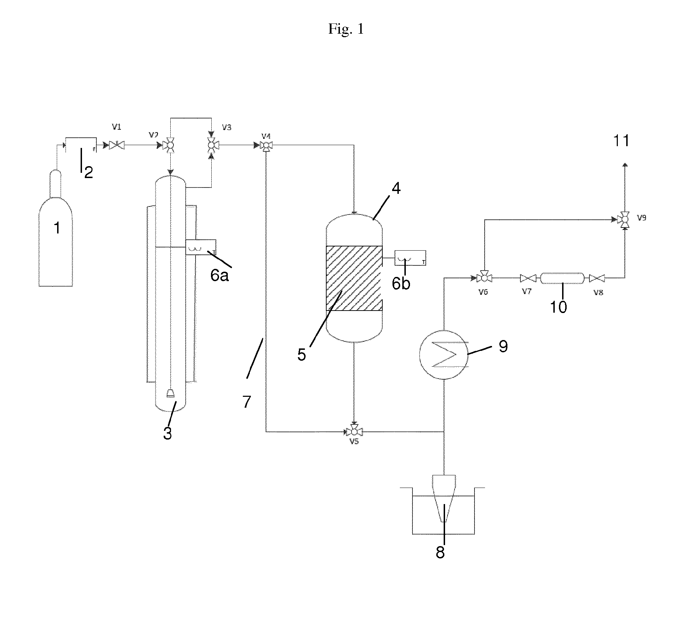

Der Versuchsaufbau der verwendeten Laborversuchsanlage ist in Figur 1 schematisch dargestellt.

Zur Förderung des Eduktes Ethanol wurde ein temperierbarer Sättiger (V = 3 l) 3 in die Apparatur integriert. Mit Hilfe dieser Konstruktion konnten bei konstantem Stickstoffvolumenstrom (5 l/h) aus der Stickstoffbombe 1, der über die Mass Flow Controller **2** und die Ventile **V1** und **V2** geregelt wurde, in Abhängigkeit von der Sättigertemperatur, die über die Temperatursteuerungseinheit **6a** geregelt wurde unterschiedliche Ethanolkonzentrationen am Eingang des Reaktors **4** realisiert werden. Die Bestimmung der eingebrachten Ethanolmenge wurde durch Nutzung des Bypasses **7** zwischen den Ventilen **V4** und **V5** unter Umgehung des Reaktors **4** und nachfolgendem Auswiegen der in der Kühlfalle **8** ausgefrorenen Ethanolmenge realisiert. Die Berechnung der geförderten Ethanolmenge erfolgte vor jedem Versuchsdurchlauf (Doppelbestimmung, zweimal über je 90 min).

Als Reaktor **4** wurde ein Quarzglasrohrreaktor mit einer Länge von 560 mm und einem Volumen von 28 ml (+/- 1 ml) genutzt. Für die katalytischen Messungen werden jeweils 500 mg Katalysator verwendet. Das Katalysatorfestbett **5** wurde im Reaktor mit Hilfe von Quarzglaswolle fixiert und wies je nach Schüttvolumen der Probe eine Höhe von 45 bis 55 mm auf.

Vor jedem katalytischen Test erfolgte im Reaktor **4** standardmäßig eine Kalzination der Probe über 5 Stunden bei 450°C in Stickstoff (5 l/h). Anschließend wird die Reaktortemperatur auf 400°C abgesenkt und die mit Hilfe des Sättigers **3** geförderte Ethanolmenge bestimmt. Der berechnete Mittelwert der geförderten Ethanolmenge galt als Re-

chenwert $m_0$ bzw. $n_0$ der eingebrachten Masse bzw. Stoffmenge in den Reaktor **4**. Die Temperatur im Reaktor wurde über die Temperatursteuerungseinheit **6b** geregelt.

Für einen Vorlauf wurde der Ethanolstrom für 30 Minuten in den Reaktor eingeleitet, die erhaltenen Produkte wurden jedoch verworfen. Erst nach dieser Vorlaufzeit begann die eigentliche katalytische Messung bei 400°C. Die Reaktionszeit (TOS, <u>T</u>ime <u>O</u>n <u>S</u>tream) belief sich auf 90 min. Nach 60 Minuten erfolgt die Analyse der Gasphase, wobei die gasförmige Probe über den Kühler **9** mit Hilfe einer Gasmaus **10** (V = 0,65 l) in den Gaschromatographen überführt wurde.

Weitere 30 Minuten später wurde die Kühlfalle **8** (V = 0,0251) ausgebaut, die darin kondensierte Flüssigphase ausgewogen und gaschromatographisch analysiert. Währenddessen wurde nach gleicher Verfahrensweise über weitere 90 Minuten eine zweite katalytische Messung bei 400°C durchgeführt. Im Anschluss daran wurde die Reaktortemperatur schrittweise (5 K/min) auf 450°C erhöht und nach Absolvierung eines erneuten Vorlaufes (30 min) ein katalytischer Test bei 450°C (90 min) durchgeführt.

Zur Bilanzierung wurden die Analysenergebnisse des zweiten Tests bei 400°C und des Tests bei 450°C herangezogen.

**Analytik und Bilanzierung**

Die Auswertung der erhaltenen Ergebnisse erfolgte über eine vollständige Massenbilanzierung. Diese setzte die Analyse aller auftretenden Verbindungen voraus. Sichergestellt wurde dies durch eine gaschromatographische Analyse, welche nach folgenden Parametern durchgeführt wurde:

| | |
|---|---|
| Gaschromatograph | HP GC 6890 |
| Säule | HP-1, 100m (unpolar) |
| Säulendurchmesser | 0,25 mm |
| Filmdicke | 0,5 $\mu$m |
| Trägergas | Helium 5.0 |
| Splitverhältnis | 100:1 |
| Detektor | Flammenionisationsdetektor |
| Injektionsvolumen (Gasprobe) | 200 $\mu$l |
| Injektionsvolumen (Flüssigprobe) | 0,5 $\mu$l |

Als Analysenergebnis wurde für die Gas- und Flüssigphase jeweils ein Gaschromatogramm erhalten. Unter Verwendung von zuvor bestimmten Responsefaktoren wurden die erhaltenen Flächen gemäß dem jeweiligen substanzspezifischen Ansprechverhalten korrigiert. Mit diesem Verfahren werden die Flächenprozente in Massenprozente umgerechnet. Da die eingebrachte Menge an Ethanol und die Masse der erhaltenen Flüssigphase bekannt war, konnte die Gesamtmasse der Gasphasenprodukte aus dieser Differenz berechnet werden. In Kombination mit den bekannten Konzentrationen der jeweiligen Substanzen in der Flüssig- und Gasphase konnte die jeweils vorliegende Masse bzw. Stoffmenge berechnet werden.

Die Berechnung des Ethanolumsatzes bzw. der Selektivitäten der jeweiligen Produkte wurde unter Berücksichtigung der jeweiligen Stöchiometriefaktoren mit Hilfe der nachfolgenden Gleichungen berechnet:

$$X = \frac{n_{Ethanol}}{n_{Ethanol,0}} \cdot 100 \ \% \qquad (1)$$

$$S = \frac{n_{Produkt}}{n_{Ethanol,0} - n_{Ethanol}} \cdot \frac{|v_A|}{v_{Produkt}} \cdot 100 \ \% \qquad (2)$$

Es wurden folgende Ergebnisse erhalten:

| Beispiel | Katalysator | WHSV | T [°C] | TOS [h] | Umsatz [%] | Selektivität Butadien [%] | Ausbeute Butadien [%] | Butadien-durchsatz [g/g*h] |
|---|---|---|---|---|---|---|---|---|
| 13 | MgO-HDK-24k | 1,16 | 400 | 3 | 93,4 | 53,0 | 49,5 | 0,57 |
| | | 1,16 | 450 | 4,5 | 98,6 | 56,2 | 55,4 | 0,64 |
| 14 | MgO-HDK-ZnO-0,25-24ki | 1,08 | 400 | 3 | 91,0 | 66,7 | 60,7 | 0,66 |
| | | 1,08 | 450 | 4,5 | 99,1 | 60,6 | 60,1 | 0,65 |
| 15 | MgO-HDK-ZnO-0,5-24ki | 1,11 | 400 | 3 | 90,4 | 64,1 | 57,9 | 0,64 |
| | | 1,11 | 450 | 4,5 | 98,2 | 57,0 | 56,0 | 0,62 |
| 16 | MgO-HDK-ZnO-5-24ki | 1,19 | 400 | 3 | 88,6 | 25,9 | 22,9 | 0,27 |
| | | 1,19 | 450 | 4,5 | 99,3 | 26,6 | 26,4 | 0,31 |
| 17 | MgO-HDK-MnO-0,5-24ki | 1,29 | 400 | 3 | 70,8 | 60,6 | 42,9 | 0,55 |
| | | 1,29 | 450 | 4,5 | 92,3 | 61,2 | 56,5 | 0,73 |
| 18 | MgO-HDK-CuO-0,5-24ki | 1,18 | 400 | 3 | 77,0 | 62,1 | 47,8 | 0,56 |
| | | 1,18 | 450 | 4,5 | 100,0 | 59,9 | 59,9 | 0,71 |
| 19 | MgO-HDK-NiO-0,5-24ki | 1,29 | 400 | 3 | 90,8 | 68,2 | 61,9 | 0,80 |
| | | 1,29 | 450 | 4,5 | 95,2 | 65,3 | 62,2 | 0,80 |
| 20 | MgO-HDK-$V_2O_5$-0,5-24ki | 1,17 | 400 | 3 | 90,0 | 65,3 30,9 | 27,8 | 0,33 |
| | | 1,17 | 450 | 4,5 | 99,0 | 24,8 | 24,6 | 0,29 |
| 21 | MgO-HDK-$TiO_2$-0,5-24ki | 1,01 | 400 | 3 | 82,2 | 57,8 | 47,5 | 0,48 |
| | | 1,01 | 450 | 4,5 | 94,2 | 57,2 | 53,9 | 0,54 |
| 22 | MgO-HDK-$Cr_2O_3$-0,5-24ki | 1,21 | 400 | 3 | 91,7 | 64,4 | 59,1 | 0,71 |
| | | 1,21 | 450 | 4,5 | 98,6 | 58,9 | 58,1 | 0,70 |
| 23 | MgO-HDK-24k (92.5:7,5) | 1,09 | 400 | 3 | 75,3 | 48,6 | 36,6 | 0,40 |
| | | 1,09 | 450 | 4,5 | 94,8 | 58,9 | 55,8 | 0,61 |
| 24 | MgO-$SiO_2$-(1:1)-0,1Na | 1,51 | 400 | 3 | 88,4 | 14,0 | 12,4 | 0,19 |
| | | 1,51 | 450 | 4,5 | 93,2 | 20,0 | 18,6 | 0,28 |

TOS = Dauer der Ethanolüberleitung (Time On Stream) WHSV = Katalysatorbelastung (Weight-Hour-Space-Velocity; Ethanolzufuhr in g/h pro g Katalysator)

**Patentansprüche**

1. Katalysator, enthaltend Sauerstoffverbindungen des Magnesiums und des Siliciums, **dadurch gekennzeichnet, dass** das molare Verhältnis von Magnesium zu Silicium von 3,5:1 bis 15:1, vorzugsweise 4:1 bis 15 :1 und besonders bevorzugt 5,5:1 bis 12,5:1 beträgt.

2. Katalysator nach Anspruch 1, **dadurch gekennzeichnet, dass** die Sauerstoffverbindungen des Magnesiums ausgewählt sind aus der Gruppe Magnesiumhydroxid und Magnesiumoxid, wobei Magnesiumoxid bevorzugt ist.

3. Katalysator nach Anspruch 1 oder 2, **dadurch gekennzeichnet, dass** die Sauerstoffverbindungen des Siliciums ausgewählt sind aus der Gruppe der kristallinen oder nicht-kristallinen Modifikationen bzw. Erscheinungsformen des Siliciumdioxids und Kieselsäuren der Formel $H_{2n+2}Si_nO_{3n+1}$, wobei n eine natürliche Zahl ist.

4. Katalysator nach Anspruch 3, **dadurch gekennzeichnet, dass** er als Sauerstoffverbindungen des Siliciums pyrogene Kieselsäuren enthält.

5. Katalysator nach einem der Ansprüche 1 bis 4, **dadurch gekennzeichnet, dass** er als Sauerstoffverbindungen des Magnesiums und Siliciums Magnesiumsilikate, wie zum Beispiel Talk und Sepiolith sowie Mineralien der Serpentingruppe enthält.

6. Katalysator nach einem der Ansprüche 1 bis 5, **dadurch gekennzeichnet, dass** er von 70 bis 100 Gew.-% Sauerstoffverbindungen des Magnesiums und des Siliciums, bevorzugt 90 bis 100 Gew.-%, besonders bevorzugt 98 bis 100 Gew.-% enthält.

7. Katalysator nach einem der Ansprüche 1 bis 6, **dadurch gekennzeichnet, dass** er weiterhin zumindest eine Übergangsmetallverbindung enthält, wobei die Übergangsmetallverbindungen vorzugsweise ausgewählt sind aus Sauerstoff-Verbindungen der Elemente Titan, wie insbesondere Titan(IV)oxid, Vanadium, wie insbesondere Vanadium(V)oxid, Molybdän, Wolfram, Mangan, wie insbesondere Mangan(II)oxid,, Kupfer, wie insbesondere Kupfer(II)oxid, Nickel, wie insbesondere Nickel(II)oxid, Zink, wie insbesondere Zink(II)oxid oder Chrom, wie insbesondere Chrom(III)oxid.

8. Katalysator nach Anspruch 7, **dadurch gekennzeichnet, dass** das molare Verhältnis von Magnesium zu Übergangsmetall von 15:1 bis 1000:1, vorzugsweise 50:1 bis 750:1, besonders bevorzugt von 75:1 bis 500:1 und ganz besonders bevorzugt von 80:1 bis 350:1 beträgt.

9. Katalysator nach einem der Ansprüche 1 bis 8, **dadurch gekennzeichnet, dass** er 0,1 Gew.-% oder weniger an Alkalimetallverbindungen berechnet auf $M_2O$ enthält, wobei M ein Alkalimetall, wie insbesondere Lithium, Natrium oder Kalium ist, vorzugsweise weniger als 0,08 Gew.-%.

10. Katalysator nach einem der Ansprüche 1 bis 9, **dadurch gekennzeichnet, dass** die spezifische Oberfläche der erfindungsgemäßen Katalysatoren 20 bis 500 $m^2$/g, vorzugsweise 20 bis 250 $m^2$/g und besonders bevorzugt 100 bis 250 $m^2$/g beträgt.

11. Katalysator nach einem der Ansprüche 1 bis 10, **dadurch gekennzeichnet, dass** das Porenvolumen gemessen nach DIN 66 133 von 0,05 bis 0,4 $cm^3$/g, vorzugsweise 0,1 bis 0,4 $cm^3$/g und besonders bevorzugt 0,13 bis 0,34 $cm^3$/g beträgt.

12. Verfahren zur Herstellung von Katalysatoren nach einem der Ansprüche 1 bis 11, **dadurch gekennzeichnet, dass** es zumindest folgende Schritte umfasst:

A) Herstellung eines Katalysatormaterials durch

i) Fällung zumindest einer im Wesentlichen wasserlöslichen Magnesiumverbindung auf zumindest eine feste Sauerstoffverbindung des Siliciums oder zumindest einer nicht festen Siliciumverbindung auf zumindest eine feste Sauerstoffverbindung des Magnesiums und/oder Magnesiumcarbonat in einem wässrigen Reaktionsmedium und Abtrennung des so erhaltenen, gefällten Katalysatormaterials vom wässrigen Reaktionsmedium
ii) Bereitstellung von Mischungen von Sauerstoffverbindungen des Magnesiums oder Magnesiumcarbonat und Sauerstoffverbindungen des Siliciums als Katalysatormaterial oder Bereitstellung von Sauerstoffverbindungen des Magnesiums und des Siliciums jeweils gemäß obiger Definition als Katalysatormaterial

B) Gegebenenfalls Vermahlung des Katalysatormaterials
C) Gegebenenfalls Herstellung von Formkörpern enthaltend das gemäß Schritt A) und/oder B) erhaltene Katalysatormaterial
D) Kalzinierung des Katalysatormaterials oder der Formkörper

**13.** Verfahren nach Anspruch 12, **dadurch gekennzeichnet dass** es wie folgt durchgeführt wird:

A) Herstellung eines Katalysatormaterials durch Vermischung von

● Kieselgelen oder pyrogener Kieselsäure oder Mischungen davon, vorzugsweise pyrogener Kieselsäure mit
● Magnesiumhydroxid, Magnesiumoxid oder Magnesiumcarbonat, vorzugsweise Magnesiumhydroxid und
● gegebenenfalls Übergangsmetallverbindungen wie vorzugsweise Übergangsmetalloxiden, -hydroxiden, -nitraten oder -carbonaten, vorzugsweise Oxide, Hydroxide, Nitrate oder Carbonate von Titan, Vanadium, Molybdän, Wolfram, Mangan, Kupfer, Nickel, Zink oder Chrom, besonders bevorzugt die Hydroxide und Oxide von Titan, Vanadium, Mangan, Kupfer, Nickel, Zink oder Chrom sowie Mangan, Zink-, Nickel und Kupfercarbonat

B) Vermahlung des gemäß Schritt A) erhaltenen Katalysatormaterials
C) Gegebenenfalls Herstellung von Formkörpern enthaltend das gemäß Schritt A) und/oder B) erhaltene Katalysatormaterial
D) Kalzinierung des Katalysatormaterials oder der Formkörper

**14.** Verfahren zur katalytischen Herstellung von 1,3-Butadien, **dadurch gekennzeichnet, dass** es in Gegenwart von Katalysator nach einem der Ansprüche 1 bis 11 durchgeführt wird.

**15.** Verfahren nach Anspruch 14, **dadurch gekennzeichnet**, das ein Ethanol enthaltender Gasstrom mit Katalysator in Kontakt gebracht wird, beispielsweise bei einer Temperatur von 200°C bis 500°C, vorzugsweise 250°C bis 475°C, weiter bevorzugt 325°C bis 475°C und besonders bevorzugt 350 bis 475°C.

Fig. 1

Nummer der Anmeldung

EP 12 18 6486

Europäisches
Patentamt

European
Patent Office

Office européen
des brevets

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | HIROO NIIYAMA ET AL: "Butadiene Formation from Ethanol over Silica-Magnesia Catalysts", BULLETIN OF THE CHEMICAL SOCIETY OF JAPAN, Bd. 45, Nr. 3, 1. Januar 1972 (1972-01-01), Seiten 655-659, XP055051156, ISSN: 0009-2673, DOI: 10.1246/bcsj.45.655 | 1-6, 10-15 | INV. B01J21/08 B01J21/10 B01J21/14 B01J23/22 B01J23/28 B01J23/30 |
| Y | * Zusammenfassung * * Experimental, "Catalysts"; Seite 655 * * Experimental, "Reaction"; Seite 655 * * Results and Discussions; Seite 655, Absatz 1 * * Abbildungen 1, 3 * * Tabellen 1, 3 * ----- -/-- | 7-9 | B01J23/34 B01J23/56 B01J23/72 B01J23/755 B01J23/78 C07C11/167 C07C1/02 C07C1/20 |

RECHERCHIERTE
SACHGEBIETE (IPC)

B01J
C07C

Der vorliegende Recherchenbericht wurde für alle Patentansprüche erstellt

| Recherchenort | Abschlußdatum der Recherche | Prüfer |
|---|---|---|
| München | 25. Januar 2013 | Kyriopoulos, Aliki |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes
    Dokument

EPO FORM 1503 03.82 (P04C03)

Europäisches Patentamt
European Patent Office
Office européen des brevets

**EUROPÄISCHER RECHERCHENBERICHT**

Nummer der Anmeldung

EP 12 18 6486

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | S. KVISLE ET AL: "Transformation of ethanol into 1,3-butadiene over magnesium oxide/silica catalysts", APPLIED CATALYSIS, Bd. 43, Nr. 1, 1. Januar 1988 (1988-01-01), Seiten 117-131, XP055051157, ISSN: 0166-9834, DOI: 10.1016/S0166-9834(00)80905-7 | 1-15 | |
| Y | * Zusammenfassung * <br> * Introduction; <br> Seite 117, Absatz 1 * <br> * Seite 118, Absatz 2 * <br> * Experimental, "Catalyst preparation"; <br> Seite 119 * <br> * Experimental, "Catalytic test"; <br> Seite 120 * <br> * Seite 122, Zeile 5 - Zeile 6 * <br> * Seite 123, Absatz 3 * <br> * Conclusion; <br> Seite 129 - Seite 130 * <br> * Abbildungen 1, 2 * <br> * Tabelle 1 * <br> ----- | 1-15 | |
| X | US 2 920 049 A (CYRIL ROMANOVSKY ET AL) 5. Januar 1960 (1960-01-05) <br> * Spalte 1, Zeile 15 - Zeile 18 * <br> * Spalte 1, Zeile 36 - Zeile 42 * <br> * Spalte 1, Zeile 55 - Zeile 57 * <br> * Spalte 1, Zeile 61 - Spalte 2, Zeile 12 * <br> * Spalte 2, Zeile 22 - Zeile 28 * <br> * Spalte 2, Zeile 41 - Zeile 42 * <br> * Spalte 3, Zeile 28 - Zeile 48 * <br> * Spalte 4, Zeile 63 - Spalte 6, Zeile 60 * <br> * Spalte 3 - Spalte 4; Tabellen * <br> ----- <br> -/-- | 1-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 25. Januar 2013 | Kyriopoulos, Aliki |

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 12 18 6486

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2 447 181 A (BUTTERBAUGH DARREL J ET AL) 17. August 1948 (1948-08-17)<br>* Spalte 1, Zeile 1 - Zeile 7 *<br>* Spalte 1, Zeile 12 - Zeile 18 *<br>* Spalte 1, Zeile 53 - Spalte 2, Zeile 25 *<br>* Spalte 2, Zeile 29 - Zeile 34 *<br>* Spalte 2, Zeile 39 - Zeile 45 *<br>* Spalte 2, Zeile 53 - Spalte 3, Zeile 37 *<br>* Spalte 3, Zeile 59 - Zeile 70 *<br>* Spalte 5 - Spalte 6; Tabellen *<br>* Spalte 6, Zeile 59 - Zeile 69 *<br>----- | 1-15 | |
| Y,D | RYUICHIRO OHNISHI ET AL: "Pronounced catalytic activity and selectivity of MgO?SiO2?Na2O for synthesis of buta-1,3-diene from ethanol",<br>JOURNAL OF THE CHEMICAL SOCIETY, CHEMICAL COMMUNICATIONS,<br>Nr. 22, 1. Januar 1985 (1985-01-01), Seite 1613, XP055051122,<br>ISSN: 0022-4936, DOI: 10.1039/c39850001613<br>* Seite 1613, linke Spalte, Zeile 5 - rechte Spalte, Zeile 14 *<br>* Seite 1614, linke Spalte, Zeile 11 - Zeile 21 *<br>* Tabelle 1 *<br>-----<br>-/-- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 25. Januar 2013 | Kyriopoulos, Aliki |

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 12 18 6486

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | B. B. CORSON ET AL: "Butadiene from Ethyl Alcohol. Catalysis in the One-and Two-Stop Processes.", INDUSTRIAL & ENGINEERING CHEMISTRY, Bd. 42, Nr. 2, 1. Februar 1950 (1950-02-01), Seiten 359-373, XP055051002, ISSN: 0019-7866, DOI: 10.1021/ie50482a039 * Zusammenfassung * * Tabellen II, IX * ----- | 1-15 | |
| A | KITAYAMA YOSKIE ET AL: "CATALYTIC ACTIVITY OF FIBROUS CLAY MINERAL SEPIOLITE FOR BUTADIENE FORMATION FROM ETHANOL", JOURNAL OF THE CHEMICAL SOCIETY, CHEMICAL COMMUNICATIONS, CHEMICAL SOCIETY. LETCHWORTH, GB, Bd. 9, 1. Januar 1981 (1981-01-01), Seiten 401-402, XP009085246, ISSN: 0022-4936, DOI: 10.1039/C39810000401 * Zusammenfassung * * Seite 401, rechte Spalte, Zeile 2 - Zeile 4 * * Seite 401, rechte Spalte, Zeile 8 - Zeile 11 * * Seite 402, rechte Spalte, Zeile 1 - Zeile 11 * * Seite 402; Tabellen * ----- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 25. Januar 2013 | Kyriopoulos, Aliki |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..............................................................................................
& : Mitglied der gleichen Patentfamilie, übereinstimmendes Dokument

EPO FORM 1503 03.82 (P04C03)

**EP 2 712 673 A1**

| Im Recherchenbericht angeführtes Patentdokument | Datum der Veröffentlichung | Mitglied(er) der Patentfamilie | Datum der Veröffentlichung |
|---|---|---|---|
| US 2920049 A | 05-01-1960 | KEINE | |
| US 2447181 A | 17-08-1948 | KEINE | |

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 331482 A, Lebedev
- PL 44585

- WO 2012015340 A

**In der Beschreibung aufgeführte Nicht-Patentliteratur**

- **MAKSHINA, E.V ; JANSSENS, W. ; SELS, B.F ; JACOBS, P.A.** Catalytic study of the conversion of ethanol into 1,3-butadiene. *Catalysis Today,* 2012
- **ERDEV et al.** *Acta Chimica, Academiae Scientiarium Hungaricae Tomus,* 1966, vol. 50, 163-166

- **OHNISHI et al.** *J. Chem. Soc., Chem. Commun.,* 1985, 1613-1614
- **Y. KITAYAMA ; K. SHIMIZU ; T. KODAMA ; S. MURAI ; T. MIZUSIMA ; M. HAYAKAWAA ; M. MU-RAOKA.** Role of intracrystalline tunnels of sepiolite for catalytic activity. *Studies in Surface Science and Catalysis,* 2002, vol. 142, 675-682